# FASCICULE DE BREVET EUROPEEN

(11) **EP 3 387 409 B1**
(45) Date de publication et mention de la délivrance du brevet: **08.01.2020**
(21) Numéro de dépôt: 16795274.6
(22) Date de dépôt: 08.11.2016
(51) Int. Cl.: G01N 1/20, G01N 1/08, G01N 1/14, G01N 33/10, G01N 1/10

(54) **DISPOSITIF D'ECHANTILLONNAGE DE SOLIDES GRANULAIRES**
VORRICHTUNG FÜR DIE SAMPLUNG VON GRANULAREN FESTSTOFFEN
DEVICE FOR SAMPLING GRANULAR SOLIDS

(30) Priorité: 10.12.2015 FR 1562153
(43) Date de publication de la demande: 17.10.2018
(73) Titulaire: AXENS, 92508 Rueil-Malmaison Cedex (FR)
(72) Inventeur: DESPAUX, Yvan, 78810 Feucherolles (FR)
(74) Mandataire: IFP Energies nouvelles
(86) Numéro de dépôt international: PCT/EP2016/076937
(87) Numéro de publication internationale: WO 2017/097524

(56) Documents cités:
- WO-A1-2011/035377
- FR-A1- 2 679 655
- US-A- 5 211 062
- GORSHTEYN A Y ET AL: "SUBSURFACE DETECTION OF ENVIRONMENTAL POLLUTANTS", INSTRUMENTATION SCIENCE & TECHNOLOGY, TAYLOR & FRANCIS INC, US, vol. 27, no. 2, 1 avril 1999 (1999-04-01), pages 111-121, XP000831275, ISSN: 1073-9149, DOI: 10.1080/10739149908085839

## Description

### Domaine technique de l'invention

La présente invention concerne un dispositif pour l'échantillonnage et l'extraction de solides granulaires, ainsi qu'un procédé pour réaliser une telle extraction.

### Etat de la technique

Des dispositifs permettant l'échantillonnage et l'extraction de solides granulaires, par exemple dans un empilement de solides granulaires (appelés également lit de solides granulaires) réparti dans une enceinte, sont connus de l'état de la technique.

Le document FR2679655 décrit une sonde de prélèvement pour l'échantillonnage et l'extraction de solides granulaires en vrac par carottage et aspiration desdits solides granulaires. La sonde de prélèvement est constituée de trois conduits juxtaposés, un conduit central et deux conduits latéraux, dans lesquels circulent à contre-courant trois flux d'airs. Dans ce dispositif, les courants gazeux circulants dans les tubes latéraux servent à équilibrer les turbulences qui sont créés lors de l'aspiration des solides granulaires dans le tube central.

Le document EP0411932 décrit une sonde pneumatique d'échantillonnage pour extraire un échantillon représentatif d'un matériau en vrac. La paroi de la sonde comprend une ouverture pour prélever l'échantillon qui est aspiré au moyen d'une pompe à vide. Ensuite, de l'air sous pression est injecté pour arrêter le prélèvement et transporter l'échantillon jusqu'à une zone de stockage. L'extrémité de la sonde qui est arrondie comprend dans sa partie interne une zone de stockage intermédiaire de l'échantillon. Cependant, ce dispositif n'est pas adapté à l'extraction de solides granulaires situés à une profondeur supérieure à un ou deux mètres, par exemple dans un silo ou dans un lit de catalyseur ou d'adsorbant compris dans un réacteur.

Le document US4072059 décrit un dispositif comprenant une sonde de prélèvement comportant plusieurs ouvertures de prélèvement d'échantillons suitées à différents niveaux de la sonde, ainsi que des moyens de succions reliés auxdites ouvertures pour prélever des échantillons dans un empilement de solides granulaires. Un tel dispositif ne permet pas non plus d'effectuer des prélèvements au-delà de deux mètres voire moins, car seule la force humaine permet d'enfoncer le dispositif dans un empilement de solides granulaires. Il est donc nécessaire comme pour le dispositif divulgué dans le document EP0411932 de décharger partiellement le silo ou le réacteur contenant le lit de catalyseur ou d'adsorbant par tranche d'un ou deux mètres pour effectuer des prélèvements à plusieurs hauteurs.

Le dispositif selon l'invention permet de remédier aux inconvénients des dispositifs de l'art antérieur en proposant un dispositif d'échantillonnage et d'extraction de solides granulaires situés dans un empilement de solides granulaires permettant un prélèvement d'échantillons à des profondeurs variables dans l'empilement, sans qu'aucune opération intermédiaire de décharge partielle de solides granulaires constituant l'empilement ne soit effectuée, et tout en préservant l'intégrité physico-chimique dudit solide granulaire.

Le dispositif selon l'invention permet d'effectuer un échantillonnage de solides granulaires en petite quantité (20 à 200 cm³) et donc dans une zone limitée avec une bonne précision, ce qui permet de déterminer l'état du solide granulaire dans ces différentes zones. Ceci permet de contrôler facilement l'état d'un solide granulaire ou de déterminer les raisons de son éventuelle dégradation via des analyses de ces prélèvements locaux.

Le dispositif selon l'invention permet de contrôler la qualité des solides granulaires et/ou leurs propriétés physico-chimiques, comme par exemple des céréales (blé, maïs, colza, riz) stockées en silos, des sables, gravillons charbons, ciments ou autres matières solides dont on veut contrôler leur qualité. Le dispositif selon l'invention permet de détecter une détérioration de la qualité de grains de céréales dans des enceintes de stockage.

Le dispositif selon l'invention est ainsi particulièrement utile pour la détection de poisons de catalyseurs ou d'adsorbants solides qui provoquent une détérioration de leur performance. Il permet de déterminer l'ampleur de la zone d'un lit de catalyseur ou d'un adsorbant concerné par cet empoisonnement.

Par exemple, le dispositif selon l'invention permet également de vérifier le bon fonctionnement d'un adsorbant via des prélèvements locaux et des analyses qui permettent de vérifier que les produits à adsorber sont effectivement retenus par ledit adsorbant.

### Objets de l'invention

Un premier objet selon l'invention concerne un dispositif pour l'échantillonnage et l'extraction d'au moins un solide granulaire selon l'objet de la revendication d'appareil 1.

Avantageusement, la distance entre les deux clapets étant comprise entre 100 et 400 mm. De préférence, les deux clapets sont pivotants sur leur bord inférieur.

Avantageusement, lesdits conduits centraux desdits tubes d'échantillonnage et de transfert sont reliés à une source de fluide gazeux.

De préférence, ledit organe de commande en fluide gazeux est apte à créer une dépressurisation dans les conduits centraux desdits tube d'échantillonnage et de transfert.

De préférence, lesdits tubes de transfert ont une longueur comprise entre 200 et 900 mm.

De préférence, lesdits tubes de transfert ont un diamètre compris entre 25 et 70 mm.

Avantageusement, ledit dispositif comprend entre 1 et 30 tubes de transfert.

De préférence, lesdits tubes d'échantillonnage et de transfert comprennent chacun un conduit latéral supplémentaire.

Avantageusement, lesdits conduits latéraux supplémentaires desdits tubes d'échantillonnage et de transfert sont connectés à une source de fluide gazeux.

De préférence, le volume de l'espace de collecte est compris entre 20 et 200 cm³.

Un autre objet selon l'invention concerne un procédé pour l'échantillonnage et l'extraction d'au moins un solide granulaire selon l'objet de la revendication méthode 12.

Un autre objet selon l'invention concerne l'utilisation d'un dispositif selon l'invention pour l'échantillonnage et l'extraction d'au moins un solide granulaire choisi parmi le groupe de solides granulaires suivant : les grains de catalyseurs, les céréales, les sables, les gravillons, les charbons, les ciments.

### Description des figures

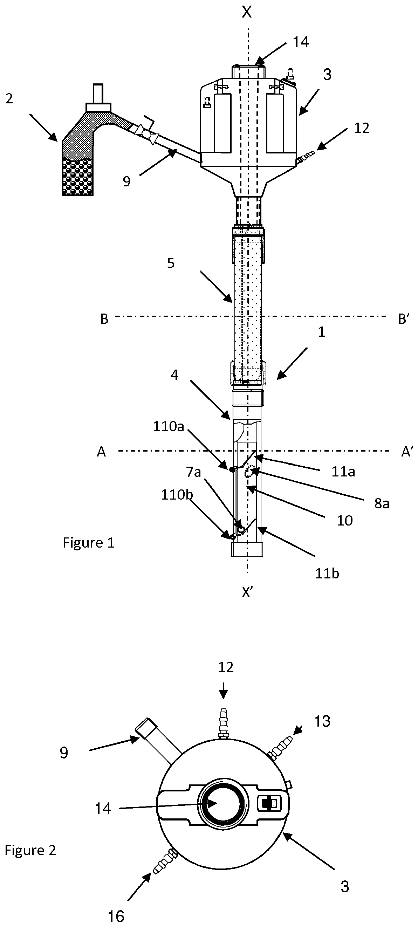

La figure 1 est une vue en coupe longitudinale du dispositif selon l'invention (un seul tube de transfert **5** est représenté sur la figure 1) comprenant une sonde de prélèvement **1**, un collecteur d'échantillon **2**, et un organe de commande **3**, ledit tube d'échantillonnage **4** comportant un espace de collecte **10** comprenant un système de retenue **11** se présentant sous la forme d'un système à deux clapet **11a**,**11b.**
La figure 2 est une vue de dessus du dispositif selon l'invention dans laquelle les entrées et sorties des fluides gazeux sont représentées.

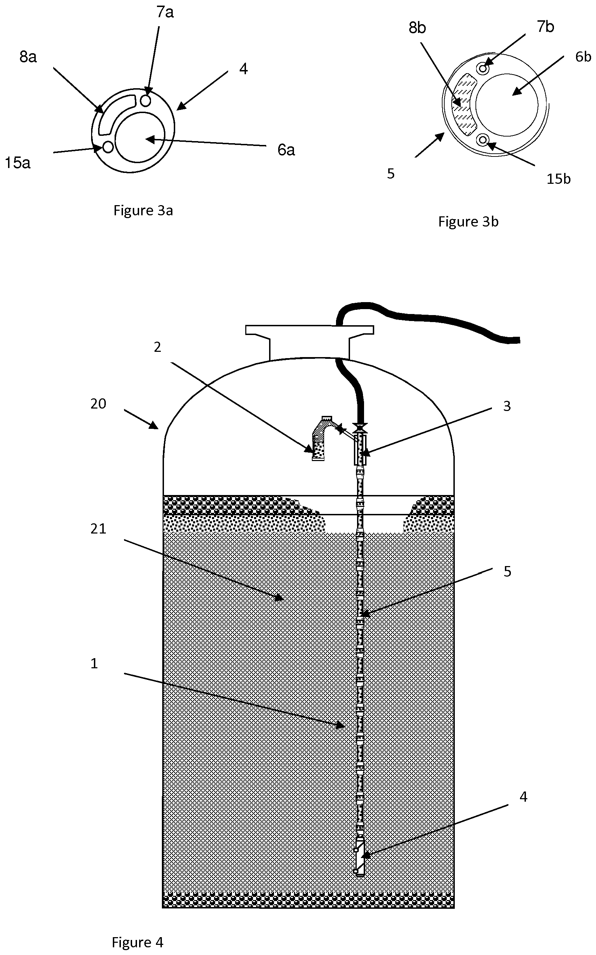

La figure 3a est une vue en coupe transversale suivant l'axe AA' représenté sur la figure 1 d'un tube d'échantillonnage **4** du dispositif selon l'invention, la figure 3b est une vue en coupe transversale suivant l'axe BB' représenté sur la figure 1 d'un tube de transfert **5** du dispositif selon l'invention.
La figure 4 illustre un dispositif selon l'invention mis en place dans un réacteur **20** comprenant un empilement de solides granulaires **21** se présentant sous la forme de grains de catalyseur. Le dispositif selon l'invention comprend une sonde de prélèvement **1**, un collecteur d'échantillon **2**, et un organe de commande **3**, ladite sonde de prélèvement comportant un tube d'échantillonnage **4** et une pluralité de tubes de transfert **5** (12 tubes de transfert sont représentés sur la figure 5).

### Description détaillée de l'invention

L'invention est maintenant décrite en référence aux figures 1 à 4 représentant un mode de réalisation du dispositif selon l'invention fourni à titre d'exemple et non limitatif.

En se reportant à la figure 1, le dispositif selon l'invention comprend une sonde de prélèvement **1**, un collecteur d'échantillon **2** et un organe de commande **3**, ledit collecteur d'échantillon **2** étant relié à ladite sonde de prélèvement **1** via une conduite de sortie **9** située sur l'organe de commande **3.** La sonde de prélèvement **1** comprend deux parties distinctes. La partie inférieure de la sonde de prélèvement **1** comprend un tube d'échantillonnage **4**, la partie supérieure de la sonde de prélèvement comprend au moins un tube de transfert **5.** Le tube d'échantillonnage **4** a pour fonction d'extraire au moins un solide granulaire se trouvant dans un empilement, le tube de transfert **5** a pour fonction de transférer le solide granulaire extrait via le tube d'échantillonnage **4** vers le collecteur d'échantillon **2.**

Les tubes d'échantillonnage **4** et de transfert **5** sont compartimentés en plusieurs conduits qui sont parallèles et étanches sur toute leur longueur. Lors de l'opération de montage du dispositif selon l'invention, les tubes d'échantillonnage **4** et de transfert **5** sont assemblés progressivement bout à bout par vissage et sont descendus verticalement ou en oblique, par exemple dans un lit catalytique (figure 4).

En se référant aux figures 1, 3a et 3b, le tube d'échantillonnage **4** comprend un conduit central **6a** et deux conduits latéraux **7a**,**8a** et ledit tube de transfert **5** comprend un conduit central **6b** et deux conduits latéraux **7b**,**8b.** Lorsque le dispositif selon l'invention est assemblé et est en fonctionnement, le conduit central **6a** du tube d'échantillonnage **4** est relié au conduit central **6b** du tube de transfert 5. De même, les conduits latéraux **7a**,**8a** du tube d'échantillonnage **4** sont reliés respectivement aux conduits latéraux **7b**,**8b** du tube de transfert **5.**

Le tube d'échantillonnage **4** comprend un espace de collecte **10** (cf. figure 1) situé dans le conduit central **6a.** Cet espace de collecte **10** comprend en outre un système de retenue **11** permettant de retenir un solide granulaire extrait d'un empilement. Le volume de l'espace de collecte **10** est compris 20 et 200 cm³, de préférence entre 50 et 150 cm³. Plus particulièrement, le système de retenue **11** se présente sous la forme d'un système à deux clapets **11**a, **11b** possédant la fonction de s'ouvrir et se fermer simultanément, par exemple à l'aide d'un vérin pneumatique ou de tout autre moyen équivalent. Les clapets sont pivotants sur leur bord inférieur **110a**,**110b.** De préférence, les bords inférieurs des clapets **110a**,**110b** sont fixés directement sur les parois du conduit central **6a** du tube d'échantillonnage **4.** Les clapets **11a**,**11b** sont disposés longitudinalement sur le tube d'échantillonnage 4, suivant l'axe AA' tel que représenté en figure 1. De préférence, la distance entre les deux clapets **11a**,**11b** est comprise entre 100 et 400 mm, de préférence entre 150 et 300 mm. Le tube d'échantillonnage **4** présente une longueur comprise entre 200 et 900 mm, de préférence entre 300 et 800 mm, et un diamètre compris entre 25 et 70 mm, de préférence 30mm et 60mm et de manière plus préférée 35 et 55 mm.

Le nombre de tubes de transfert **5** peut être variable en fonction de l'utilisation envisagée et notamment de la profondeur d'analyse souhaitée. De préférence le nombre de tubes de transfert **5** est compris entre 1 et 30, et de manière préférée entre 1 et 20. Les tubes de transfert **5** présentent une longueur comprise entre 200 et 900 mm, de préférence entre 300 et 800 mm, et un diamètre compris entre 25 et 70 mm, de préférence 30mm et 60mm et de manière plus préférée 35 et 55 mm. Dans un mode de réalisation particulier selon l'invention, chaque tube de transfert **5** peut avoir un diamètre différent entre sa partie supérieure et sa partie inférieure. Avantageusement, les tubes de transfert **5** sont tous identiques et indépendants les uns des autres.

Les tubes de transfert **5** sont alignés verticalement et sont raccordés entre eux. Au moins un tube de transfert **5** est raccordé à l'organe de commande **3**, et au moins un tube de transfert est raccordé au tube d'échantillonnage **4.** Dans le mode de réalisation particulier pour lequel un seul tube de transfert **5** est utilisé, ledit tube de transfert est raccordé à la fois au tube d'échantillonnage **4** et à l'organe de commande **3.**
Les tubes d'échantillonnage **4** et de transfert **5** peuvent être raccordés entre eux par vissage étanche au fur et à mesure de la descente de la sonde de prélèvement **1** dans l'empilement de solides granulaires. Cela permet d'atteindre, selon la profondeur préalablement déterminée la position du point à échantillonner. La partie inférieure l'organe de commande **3** et la partie supérieure du tube d'échantillonnage **4** présentent de préférence des formes et des dimensions compatibles avec celles des tubes de transfert **5** pour leur raccordement.

Avantageusement, l'organe de commande **3** est apte à créer une dépressurisation dans les conduits centraux **6a**,**6b** desdits tube d'échantillonnage **4** et de transfert **5.** La formation d'une dépression gazeuse dans les conduits centraux **6a**,**6b** desdits tube d'échantillonnage **4** et de transfert **5** permet de faciliter la descente du dispositif selon l'invention dans la profondeur de l'empilement de solides granulaires.

Selon l'invention, les conduits latéraux **7a**,**8a** des tubes d'échantillonnage **4** et les conduits latéraux **7b**,**8b** des tubes de transfert **5** sont connectés à une source de fluide gazeux **12**, tel que de l'air ou un gaz inerte, via l'organe de commande **3** (cf. figure 2) qui est apte à extraire un solide granulaire, lorsque ce dernier se trouve retenu dans l'espace de collecte **10** du tube d'échantillonnage **4,** par les conduits latéraux **8a** puis **8b** desdits tubes d'échantillonnage **4** et de transfert **5.**

Dans un mode de réalisation particulier selon l'invention, le dispositif peut comprendre en outre un outil pneumatique vibrant (non représenté sur les figures), ayant pour fonction de faciliter la descente et/ou la remontée de la sonde de prélèvement **1** dans un empilement de solides granulaires. En particulier, lorsque les solides granulaires sont des grains de catalyseurs, la sonde de prélèvement **1** peut présenter des difficultés de pénétration dues par exemple au cokage dudit catalyseur ou à une agglomération partielle desdits grains de catalyseur. L'outil pneumatique vibrant peut être fixé directement sur le tube d'échantillonnage **4** et être alimenté par une source en fluide gazeux **16**, tel que de l'air ou un gaz inerte, traversant l'organe de commande **3** (cf. figure 2) puis via les conduits latéraux supplémentaires **15a** et **15b** desdits tubes d'échantillonnage **4** et de transfert **5** respectivement (cf. figures 3a et 3b). Plus particulièrement, l'outil pneumatique vibrant peut se présenter sous la forme deux vérins pneumatiques latéraux (non représentés sur les figures).

Le dispositif selon l'invention peut être utilisé pour l'extraction ou l'échantillonnage d'au moins un solide granulaire se trouvant dans un empilement de solides granulaires. Selon l'invention, le procédé comprend au moins les étapes suivantes :
a) on insère ladite sonde de prélèvement **1** dans un empilement de solides granulaires jusqu'à une profondeur préalablement déterminée ;
b) on extrait au moins un solide granulaire de l'empilement et on retient ledit solide granulaire dans l'espace de collecte **10** dudit tube d'échantillonnage **4** au moyen du système de retenue **11** ;
c) on injecte un fluide gazeux sous pression **12**, tel que de l'air ou un gaz inerte, dans les conduits latéraux **7a**,**7b** desdits tubes d'échantillonnage **4** et de transfert **5** pour extraire ledit solide granulaire extrait et retenu à l'étape b) ;
d) on récupère ledit solide granulaire dans ledit collecteur d'échantillon **2.**

Les étapes du procédé selon l'invention sont décrites en détail ci-après.

### Etape a)

La descente progressive du dispositif selon l'invention dans un empilement de solides granulaires est réalisé par aspiration sous vide via l'organe de commande **3** desdits solides granulaires passant dans les conduits centraux **6a**,**6b** des tubes d'échantillonnage **4** et de transfert **5**, conjuguée à une poussée progressive manuelle de l'opérateur.

Lors de la descente de la sonde de prélèvement **1** dans le lit de solides granulaires, le système de retenue **11** du tube d'échantillonnage **4** n'est pas activé. Lorsque le système de retenue **11** se présente sous la forme d'un système à deux clapets **11a** et **11b**, les deux clapets sont en position d'ouverture, i.e. les solides granulaires peuvent circuler librement dans l'espace de collecte **10** et le canal central **6a** du tube d'échantillonnage **4.**

A titre d'illustration, pour des tubes d'échantillonnage **4** et de transfert **5** de 500 mm de longueur et de 50 mm de diamètre, environ 1,5 litres de solides granulaires par mètre de tubes **4** ou **5** traversent les conduits centraux **6a** et **6b** desdits tubes d'échantillonnage **4** et de transfert **5** puis sont évacués du dispositif via la sortie **14** située sur l'organe de commande **3.** La sortie **14** peut être relié à un aspirateur (non représenté sur les figures) situé à l'extérieur du réacteur **20** (cf. figure 4), relié aux canaux centraux **6a** et **6b** via l'organe de commande **3.**

Le tube d'échantillonnage **4** et les tubes de transfert **5** sont assemblés au fur et à mesure de la descente de la sonde de prélèvement **1** dans l'empilement de solides granulaires. Le débit d'aspiration des solides granulaires peut être régulé au moyen d'un vanne d'aspiration (non représentée sur les figures). La sonde de prélèvement **1** descend ainsi dans l'empilement de solides granulaires jusqu'à une profondeur préalablement déterminée.

### Etape b)

L'étape b) du procédé selon l'invention consiste à extraire et retenir au moins un solide granulaire de l'empilement. L'aspiration sous vide des solides granulaires via l'organe de commande **3** est stoppée et le système de retenue **11** du tube d'échantillonnage **4** est activé. Lorsque le système de retenue **11** se présente sous la forme d'un système à deux clapets **11a** et **11b**, lesdits clapets se mettent en position de fermeture ; le solide granulaire compris dans l'espace de collecte **10** est retenu dans le tube d'échantillonnage **4.** Les bords inférieurs **110a** et **110b** des clapets **11a** et **11b**, fixés sur la paroi du conduit central **6a** du tube d'échantillonnage **4**, pivotent autour de leur axe de rotation, se traduisant par le passage des clapets **11a** et **11b** de la position verticale à une position horizontale.

Le maintien des clapets en position horizontale peut être amélioré par l'injection d'un flux gazeux **13** descendant, tel que de l'air ou un gaz inerte, injecté via l'organe de commande **3** (cf. figure 2) dans les conduits centraux **6a** et **6b** des tubes d'échantillonnage **4** et de transfert **5.**

Les deux clapets **11a** et **11b** en position de fermeture renferment l'échantillon de solide granulaire. L'échantillon de solide granulaire représente un volume généralement compris entre 20 et 300 cm³, de préférence entre 30 et 250 cm³.

### Etape c)

L'étape c) du procédé selon l'invention consiste en l'envoi de l'échantillon de solide granulaire retenu dans l'espace de collecte **10** vers le collecteur d'échantillon **2.** Lors de cette étape, on injecte un fluide gazeux sous pression **12**, tel que de l'air ou un gaz inerte, dans les conduits latéraux **7a**,**7b** desdits tubes d'échantillonnage **4** et de transfert **5** pour extraire ledit solide granulaire extrait et retenu à l'étape b). Plus particulièrement, la remontée de l'échantillon de solide granulaire vers le collecteur **2** est assurée par l'injection du fluide gazeux sous pression **12** descendant dans les conduits latéraux **7a** et **7b** des tubes d'échantillonnage **4** et de transfert **5**, le conduit latéral **7a** du tube d'échantillonnage **4** étant relié au conduit central **6a** dudit tube **4** au-dessus de la base du clapet inférieur **11a**. Le fluide gazeux pousse ainsi l'échantillon de solide granulaire qui s'engage dans le conduit latéral **8a** du tube d'échantillonnage **4** dont l'ouverture est située juste en-dessous du clapet supérieur **11b**. L'échantillon de solide granulaire circule dans les conduits latéraux **8a** et **8b** des tubes d'échantillonnage **4** et de transfert **5**, qui sont reliés de façon étanche au collecteur d'échantillon **2.**

Le procédé selon l'invention peut en outre comprendre des étapes supplémentaires, par exemple pour réaliser une deuxième extraction d'un solide granulaire dans l'empilement. Après l'étape d'extraction d'au moins un échantillon de solides granulaires, les tubes d'échantillonnage **4** et de transfert **5** peuvent être remontés par mise sous pression des conduits centraux **6a** et **6b** desdits tubes, avec simultanément une poussée manuelle de la sonde de prélèvement **1** vers le haut.

Le dispositif selon l'invention, ainsi que le procédé tel que décrit précédemment utilisant un tel dispositif, s'applique à tout type de solides granulaires, comme par exemple des céréales (blé, maïs, colza, riz) stockées en silos, des sables, gravillons charbons, ciments ou autres matières solides dont on veut contrôler la qualité des solides granulaires et/ou leurs propriétés physico-chimiques. Plus particulièrement, l'invention s'applique à l'échantillonnage et/ou l'extraction de solides granulaires de type grains de catalyseurs disposés dans un réacteur, par exemple dans un réacteur chimique, en vue de réaliser différentes analyses et/ou tests physico-chimiques.

## Revendications

1. Dispositif pour l'échantillonnage et l'extraction d'au moins un solide granulaire dans un empilement de solides granulaires, ledit dispositif comprenant une sonde de prélèvement (1), un collecteur d'échantillon (2) relié à ladite sonde de prélèvement (1), et un organe de commande (3), ladite sonde de prélèvement (1) comportant un tube d'échantillonnage (4) apte à extraire ledit solide granulaire dudit empilement, ledit tube d'échantillonnage (4) étant relié à au moins un tube de transfert (5) apte à transférer ledit solide granulaire extrait via ledit tube d'échantillonnage (4) vers le collecteur d'échantillon (2), ledit tube d'échantillonnage (4) et ledit tube de transfert (5) comprenant chacun au moins un conduit central et au moins deux conduits latéraux, ledit conduit central (6a) et lesdits conduits latéraux (7a,8a) dudit tube d'échantillonnage (4) étant relié respectivement au conduit central (6b) et aux conduits latéraux (7b,8b) dudit tube de transfert (5), ledit conduit central (6a) dudit tube d'échantillonnage (4) étant en outre relié aux conduits latéraux (7a,8a) dudit tube d'échantillonnage (4) ;
**caractérisé en ce que** ledit conduit central (6a) dudit tube d'échantillonnage (4) comprend un espace de collecte (10) comprenant un système de retenue (11) dudit solide granulaire, et **en ce que** lesdits conduits latéraux (7a,7b) desdits tubes d'échantillonnage (4) et de transfert (5) sont connectés à une source de fluide gazeux (12) apte à extraire ledit solide granulaire retenu dans l'espace de collecte (10) par les conduits latéraux (8a,8b) desdits tubes d'échantillonnage (4) et de transfert (5), et **en ce que** ledit système de retenue (11) du tube d'échantillonnage (4) se présente sous la forme d'un système de deux clapets (11a, 11b) disposés de part et d'autre de l'espace de collecte (10).

2. Dispositif selon la revendication 1, **caractérisé en ce que** la distance entre les deux clapets (11a,11b) étant comprise entre 100 et 400 mm.

3. Dispositif selon les revendications 1 ou 2, **caractérisé en ce que** les deux clapets (11a,11b) sont pivotants sur leur bord inférieur (110a,110b).

4. Dispositif selon l'une quelconque des revendications 1 à 3, **caractérisé en ce que** lesdits conduits centraux (6a,6b) desdits tubes d'échantillonnage (4) et de transfert (5) sont reliés à une source de fluide gazeux (13).

5. Dispositif selon l'une quelconque des revendications 1 à 4, **caractérisé en ce que** ledit organe de commande (3) en fluide gazeux est apte à créer une dépressurisation dans les conduits centraux (6a,6b) desdits tube d'échantillonnage (4) et de transfert (5).

6. Dispositif selon l'une quelconque des revendications 1 à 5, **caractérisé en ce que** lesdits tubes de transfert (5) ont une longueur comprise entre 200 et 900 mm.

7. Dispositif selon l'une quelconque des revendications 1 à 6, **caractérisé en ce que** lesdits tubes de transfert (5) ont un diamètre compris entre 25 et 70 mm.

8. Dispositif selon l'une quelconque des revendications 1 à 7, **caractérisé en ce que** ledit dispositif comprend entre 1 et 30 tubes de transfert (5).

9. Dispositif selon l'une quelconque des revendications 1 à 8, **caractérisé en ce que** lesdits tubes d'échantillonnage (4) et de transfert (5) comprennent chacun un conduit latéral supplémentaire.

10. Dispositif selon la revendication 9, **caractérisé en ce que** lesdits conduits latéraux supplémentaires desdits tubes d'échantillonnage (4) et de transfert (5) sont connectés à une source de fluide gazeux.

11. Dispositif selon l'une quelconque des revendications 1 à 10, **caractérisé en ce que** le volume de l'espace de collecte (10) est compris entre 20 et 200 cm³.

12. Procédé pour l'échantillonnage et l'extraction d'au moins un solide granulaire au moyen d'un dispositif selon l'une quelconque des revendications 1 à 11, comprenant les étapes suivantes :
a) on insère ladite sonde de prélèvement (1) dans un empilement de solides granulaires jusqu'à une profondeur préalablement déterminée ;
b) on extrait au moins un solide granulaire de l'empilement et on le retient dans l'espace de collecte (10) dudit tube d'échantillonnage (4) au moyen du système de retenue (11);
c) on injecte un fluide gazeux sous pression (12) dans les conduits latéraux (7a,7b) desdits tubes d'échantillonnage (4) et de transfert (5) pour extraire ledit solide granulaire extrait et retenu à l'étape b) ;
d) on récupère ledit solide granulaire dans ledit collecteur d'échantillon (2).

13. Utilisation d'un dispositif selon l'une quelconque des revendications 1 à 11 pour l'échantillonnage et l'extraction d'au moins un solide granulaire choisi parmi le groupe de solides granulaires suivant : les grains de catalyseurs, les céréales, les sables, les gravillons, les charbons, les ciments.

## Patentansprüche

1. Vorrichtung zur Probenahme und Extraktion von mindestens einem körnigen Feststoff in einer Packung körniger Feststoffe, wobei die Vorrichtung eine Probenahmesonde (1), einen mit der Probenahmesonde (1) verbundenen Probensammler (2) und ein Steuerelement (3) umfasst, wobei die Probenahmesonde (1) ein Probenahmerohr (4) aufweist, das in der Lage ist, den körnigen Feststoff aus der Packung zu extrahieren, wobei das Probenahmerohr (4) mit mindestens einem Überführungsrohr (5) verbunden ist, das in der Lage ist, den körnigen Feststoff, der über das Probenahmerohr (4) extrahiert wurde, zum Probensammler (2) zu überführen, wobei das Probenahmerohr (4) und das Überführungsrohr (5) jeweils mindestens einen mittigen Kanal und mindestens zwei seitliche Kanäle umfassen, wobei der mittige Kanal (6a) und die seitlichen Kanäle (7a, 8a) des Probenahmerohrs (4) jeweils mit dem mittigen Kanal (6b) und mit den seitlichen Kanälen (7b, 8b) des Überführungsrohrs (5) verbunden sind, wobei der mittige Kanal (6a) des Probenahmerohrs (4) zusätzlich mit den seitlichen Kanälen (7a, 8a) des Probenahmerohrs (4) verbunden ist;
**dadurch gekennzeichnet, dass** der mittige Kanal (6a) des Probenahmerohrs (4) eine Sammelkammer (10) umfasst, die ein System (11) zum Zurückhalten des körnigen Feststoffs umfasst, und dadurch, dass die seitlichen Kanäle (7a, 7b) des Probenahmerohrs (4) und des Überführungsrohrs (5) mit einer Quelle eines gasförmigen Fluids (12) verbunden sind, die in der Lage ist, den in der Sammelkammer (10) zurückgehaltenen körnigen Feststoff über die seitlichen Kanäle (8a, 8b) des Probenahmerohrs (4) und des Überführungsrohrs (5) zu extrahieren, und dadurch, dass das Rückhaltesystem (11) des Probenahmerohrs (4) die Form eines Systems mit zwei Klappen (11a, 11b) aufweist, die beiderseits der Sammelkammer (10) angeordnet sind.

2. Vorrichtung nach Anspruch 1, **dadurch gekennzeichnet, dass** der Abstand zwischen den beiden Klappen (11a, 11b) im Bereich zwischen 100 und 400 mm liegt.

3. Vorrichtung nach Anspruch 1 oder 2, **dadurch gekennzeichnet, dass** die beiden Klappen (11a, 11b) an ihrer Unterkante (110a, 110b) schwenkbar sind.

4. Vorrichtung nach einem der Ansprüche 1 bis 3, **dadurch gekennzeichnet, dass** die mittigen Kanäle (6a, 6b) des Probenahmerohrs (4) und des Überführungsrohrs (5) mit einer Quelle für gasförmiges Fluid (13) verbunden sind.

5. Vorrichtung nach einem der Ansprüche 1 bis 4, **dadurch gekennzeichnet, dass** die Steuereinrichtung (3) für gasförmige Fluide in der Lage ist, in den mittigen Kanälen (6a, 6b) des Probenahmerohrs (4) und des Überführungsrohrs (5) einen Unterdruck zu erzeugen.

6. Vorrichtung nach einem der Ansprüche 1 bis 5, **dadurch gekennzeichnet, dass** die Überführungsrohre (5) eine Länge im Bereich von 200 bis 900 mm aufweisen.

7. Vorrichtung nach einem der Ansprüche 1 bis 6, **dadurch gekennzeichnet, dass** die Überführungsrohre (5) einen Durchmesser im Bereich von 25 bis 70 mm aufweisen.

8. Vorrichtung nach einem der Ansprüche 1 bis 7, **dadurch gekennzeichnet, dass** die Vorrichtung zwischen 1 und 30 Überführungsrohre (5) aufweist.

9. Vorrichtung nach einem der Ansprüche 1 bis 8, **dadurch gekennzeichnet, dass** das Probenahmerohr (4) und das Überführungsrohr (5) jeweils einen zusätzlichen seitlichen Kanal aufweisen.

10. Vorrichtung nach Anspruch 9, **dadurch gekennzeichnet, dass** die zusätzlichen seitlichen Kanäle des Probenahmerohrs (4) und des Überführungsrohrs (5) mit einer Quelle für gasförmiges Fluid verbunden sind.

11. Vorrichtung nach einem der Ansprüche 1 bis 10, **dadurch gekennzeichnet, dass** das Volumen der Sammelkammer (10) im Bereich von 20 und 200 cm³ liegt

12. Verfahren zur Probenahme und Extraktion von mindestens einem körnigen Feststoff durch eine Vorrichtung nach einem der Ansprüche 1 bis 11, umfassend die folgenden Schritte:
a) Einführen der Probenahmesonde (1) in eine Packung körniger Feststoffe bis zu einer vorbestimmten Tiefe;
b) Extrahieren mindestens eines körnigen Feststoffs aus der Packung und Zurückhalten des Feststoffs in der Sammelkammer (10) des Probenahmerohrs (4) mithilfe des Rückhaltesystems (11);
c) Injizieren eines gasförmigen Fluids (12) unter Druck in die seitlichen Kanäle (7a, 7b) des Probenahmerohrs (4) und des Überführungsrohrs (5), um den in Schritt b) extrahierten und zurückgehaltenen körnigen Feststoff zu extrahieren;
d) Gewinnen des körnigen Feststoffs in dem Probensammler (2).

13. Verwendung einer Vorrichtung nach einem der Ansprüche 1 bis 11 zur Probenahme und Extraktion mindestens eines körnigen Feststoffs, ausgewählt aus der folgenden Gruppe der körnigen Feststoffe: Katalysatorkörner, Getreide, Sand, Splitt, Holzkohle und Zemente.

## Claims

1. A device for the sampling and extraction of at least one granular solid in a pack of granular solids, said device comprising a sampling probe (1), a sample collector (2) connected to said sampling probe (1), and a control means (3), said sampling probe (1) comprising a sampling tube (4) which is capable of extracting said granular solid from said pack, said sampling tube (4) being connected to at least one transfer tube (5) which is capable of transferring said extracted granular solid via said sampling tube (4) to the sample collector (2), said sampling tube (4) and said transfer tube (5) each comprising at least one central conduit and at least two lateral conduits, said central conduit (6a) and said lateral conduits (7a, 8a) of said sampling tube (4) being respectively connected to the central conduit (6b) and to the lateral conduits (7a, 7b) of said transfer tube (5), said central conduit (6a) of said sampling tube (4) additionally being connected to the lateral conduits (7a, 8a) of said sampling tube (4);
**characterized in that** said central conduit (6a) of said sampling tube (4) comprises a collection chamber (10) comprising a system (11) for retaining said granular solid, and **in that** said lateral conduits (7a, 7b) of said sampling tube (4) and transfer tube (5) are connected to a source of gaseous fluid (12) which is capable of extracting said granular solid retained in the collection chamber (10) via the lateral conduits (8a, 8b) of said sampling tube (4) and transfer tube (5), and **characterized in that** said system (11) for retaining the sampling tube (4) is in the form of a system with two flaps (11a, 11b) disposed either side of the collection chamber (10).

2. The device as claimed in claim 1, **characterized in that** the distance between the two flaps (11a, 11b) is in the range 100 to 400 mm.

3. The device as claimed in claim 1 or claim 2, **characterized in that** the two flaps (11a, 11b) are pivoted on their lower edge (110a, 110b).

4. The device as claimed in any one of claims 1 to 3, **characterized in that** said central conduits (6a, 6b) of said sampling tube (4) and transfer tube (5) are connected to a source of gaseous fluid (13).

5. The device as claimed in any one of claims 1 to 4, **characterized in that** said gaseous fluid control means (3) is capable of generating a partial vacuum in the central conduits (6a, 6b) of said sampling tube (4) and transfer tube (5).

6. The device as claimed in any one of claims 1 to 5, **characterized in that** said transfer tubes (5) have a length in the range 200 to 900 mm.

7. The device as claimed in any one of claims 1 to 6, **characterized in that** said transfer tubes (5) have a diameter in the range 25 to 70 mm.

8. The device as claimed in any one of claims 1 to 7, **characterized in that** said device comprises in the range 1 to 30 transfer tubes (5).

9. The device as claimed in any one of claims 1 to 8, **characterized in that** said sampling tube (4) and transfer tube (5) each comprise a supplemental lateral conduit.

10. The device as claimed in claim 9, **characterized in that** said supplemental lateral conduits of said sampling tube (4) and transfer tube (5) are connected to a source of gaseous fluid.

11. The device as claimed in any one of claims 1 to 10, **characterized in that** the volume of the collection chamber (10) is in the range 20 to 200 cm³.

12. A method for sampling and extracting at least one granular solid by means of a device as claimed in any one of claims 1 to 11, comprising the following steps:
a) inserting said sampling probe (1) into a pack of granular solids to a predetermined depth;
b) extracting at least one granular solid from the pack and retaining it in the collection chamber (10) of said sampling tube (4) by means of a retaining system (11);
c) injecting a gaseous fluid (12) under pressure into the lateral conduits (7a, 7b) of said sampling tube (4) and transfer tube (5) in order to extract said granular solid which has been extracted and retained in step b);
d) recovering said granular solid in said sample collector (2).

13. Use of a device as claimed in any one of claims 1 to 11, for the sampling and extraction of at least one granular solid selected from the following group of granular solids: grains of catalyst, cereals, sand, grit, charcoal, and cements.
